## Europäisches Patentamt

⑲ ))) **European Patent Office**

**Office européen des brevets**

(11) Numéro de publication: **0 124 461**

**B1**

(12) **FASCICULE DE BREVET EUROPÉEN**

(45) Date de publication du fascicule du brevet: **18.03.87**

(21) Numéro de dépôt: **84440016.8**

(22) Date de dépôt: **10.04.84**

(51) Int. Cl.⁴: **A 61 L 2/18, G 02 C 13/00**

(54) Procédé pour le nettoyage et la décontamination de lentilles de contact et composition pour sa mise en oeuvre.

(30) Priorité: **20.04.83 FR 8306608**

(43) Date de publication de la demande:
**07.11.84 Bulletin 84/45**

(45) Mention de la délivrance du brevet:
**18.03.87 Bulletin 87/12**

(84) Etats contractants désignés:
**AT BE CH DE GB IT LI LU NL SE**

(56) Documents cité:
**FR-A-2 226 677**
**FR-A-2 256 767**
**FR-A-2 424 311**
**GB-A-2 072 371**
**GB-A-2 094 992**
**US-A-3 908 680**

(73) Titulaire: **Laboratoires P.O.S. Société Anonyme dite:, 21 rte de Lapoutroie, F-68240 Kaysersberg (FR)**

(72) Inventeur: **Andermann, Guy, 5 rue des Jonquilles, F-68000 Colmar (FR)**
Inventeur: **Zimmermann, Georges Daniel, 38 rue de Lattre de Tassigny, F-67300 Schiltigheim (FR)**
Inventeur: **Arnold, Nöel, 105 rue de la Semm, F-68000 Colmar (FR)**
Inventeur: **Pabst, Jean-Yves, 14 rue Kentzinger, F-67600 Selestat (FR)**
Inventeur: **Stamm, André, Rue des Olives Griesheim près Molsheim, F-67210 Obernai (FR)**

(74) Mandataire: **Cuer, André, CABINET CUER 30, rue de Léningrad, F-75008 Paris (FR)**

## Description

La présente invention a pour objet un procédé pour le nettoyage et la décontamination de lentilles de contact, ainsi que des compositions pour la mise en oeuvre de ce procédé.

Le procédé de l'invention est applicable à la plupart des lentilles de contact connues, par exemple en polyméthacrylate, en hydroxyéthylméthacrylate, en acétobutyrate de cellulose etc... Il est également applicable à la décontamination des étuis de rangement des lentilles et des embouts de prothèses auditives.

Des procédés de ce type, ayant pour but de décontaminer les lentilles par élimination des micro-organismes indésirables ainsi que de les nettoyer, sont déjà connus, et de nombreuses compositions ont été proposées à ces fins.

On a suggéré, notamment, le nettoyage des lentilles par une solution aqueuse d'un hypohalogénite alcalin ou alcalino-terreux ou composé en libérant, avec lavage ultérieur à l'eau et addition éventuelle d'un agent réducteur (GB-A-2.072.371) ou encore à l'aide, en phases successives, de deux solutions aqueuses de composés à oxygène actif tel que peroxyde, percarbonate alcalin ou autre, dont l'une est acide et l'autre basique, avec traitement ultérieur par un détergent non ionique et lavage à l'eau (US-A 3.908.680).

Selon un procédé analogue au brevet anglais susvisé, le brevet FR-A-2.265.767 décrit un procédé de stérilisation de lentilles cornéennes hydrophiles, caractérisé en ce qu'il comprend la mise en contact de la lentille avec une solution d'un composé libérant un hypochlorite non toxique en quantité suffisant à la destruction des micro-organismes pathogènes présents, puis la réduction de l'hypochlorite en chlorure par un agent réducteur non toxique dont la quantité suffit à la réduction de tout l'hypochlorite.

Toutefois, ces procédés à l'hypochlorite et agent réducteur demeurent inefficaces sur un certain nombre de germes pathogènes et sont sans action sur les dépôts protéiniques responsables de "voiles" qui troublent la transparence de la lentille. Par ailleurs, la technique du brevet américain susvisé est une neutralisation acido-basique qui exige des traitements ultérieurs de nettoyage et rinçage.

La présente invention permet de remédier à ces inconvénients, c'est-à-dire d'assurer simultanément la décontamination complète et un nettoyage efficace des lentilles.

A cet effet l'invention vise un procédé pour la stérilisation et le nettoyage des lentilles de contact du type consistant à traiter la lentille par un premier agent, de caractère oxydant, ayant pour effet de détériorer les germes présents sur la lentille, puis par un second agent, de caractère réducteur, donnant naissance par réaction avec le premier agent à des sous-produits de dégradation inoffensifs pour l'oeil, le procédé étant caractérisé en ce que l'agent oxydant est choisi parmi les produits capables de libérer de l'eau oxygénée in situ alors que l'agent réducteur est choisi parmi les produits aptes à générer de l'acide hypochloreux en solution aqueuse et stables à l'état sec, les proportions respectives d'agent oxydant et d'agent réducteur étant d'environ 0,005% à 5%, et 0,02 % à 6 % en poids des substances mises en jeu.

De manière préférentielle le composé oxydant du système oxydo-réducteur sera de l'eau oxygénée libérée in situ par mise en solution d'un précurseur constitué par le premier ou le second agent.

Les systèmes oxydo-réducteurs de l'invention assurent, par une même opération, la décomposition progressive du peroxyde d'hydrogène d'après la réaction suivante :

$$2 H_2O_2 + \text{Réducteur} \rightarrow 2 H_2O + 2 (O) \rightarrow O_2$$

La lentille de contact est ainsi efficacement nettoyée et décontaminée par exposition à l'action du peroxyde d'hydrogène pendant une durée adéquate. Cependant, la lentille traitée ne doit pas nécessairement être retirée du récipient dans lequel le traitement a été effectué car le réducteur présent au départ agit de façon à décomposer $H_2O_2$ en l'éliminant ainsi par transformation en produits réactionnels inoffensifs, eau et oxygène naissant. Ce dernier, naturellement, a un effet nettoyant et une action de décontamination supplémentaire.

On notera que la quantité de $H_2O_2$ que l'on retrouve dans le milieu après la décontamination et avant la réduction est quasiment identique à la quantité initiale présente dans le milieu.

Des oxydants appropriés sont par exemple le peroxyde d'urée, les perphosphates, les perborates, les percarbonates, et les persulfates de métaux alcalins, le peroxyde d'urée étant le composé préféré selon l'invention.

La quantité d'oxydant qui doit être utilisée peut varier depuis environ 0,005 % jusqu'à 5 %, et de préférence d'environ 0,01 % à 0,1 %.

Par ailleurs le composé réducteur pourra être tout hypochlorite ou tout agent chloré donnant naissance à des anions hypochlorite au moment de l'emploi en milieu aqueux.

L'hypochlorite peut être tout hypochlorite minéral ou organique de formule générale $A(OCl)_x$ dans laquelle A représente un métal ou un groupe organique, par exemple un métal alcalin tel que le sodium, le potassium et le lithium, un métal alcalino-terreux tel que le baryum, la magnésium et le calcium, un hypochlorite d'alkyle, par exemple de tertio-butyle, de tertio-amyle, d'éthyle, de méthyle, de propyle, d'isopropyle et analogues, et $\underline{x}$ représente la charge absolue du substituant minéral ou organique. Ainsi, on peut utiliser par exemple de l'hypochlorite de sodium, de l'hypochlorite de potassium, de l'hypochlorite de lithium, de l'hypochlorite de calcium, de l'hypochlorite de tertio-butyle ou de l'hypochlorite de tertio-amyle.

Au lieu d'ajouter l'hypochlorite tel quel, on peut utiliser des matières qui donnent de l'acide hypochloreux en solution aqueuse et qui sont stables lorsqu'on les conserve à l'état sec. Ces matières sont, par exemple la N,N-dichlorotaurine (sel de sodium), la N,N-dichloro-bêta-alanine, l'acide N,N'-dichloroisocyanurique (sel de potassium), l'acide N,N'dichloroisocyanurique (sel de sodium), la N-chlorurée, la N,N'-dichloro-2,5-pipérazinedione, la N,N'-1,3-dichloro-5,5-diméthylhydantoïne, l'acide trichloroisocyanurique, le N-

2

chlorosyccinamide, la N,N'-dichloroglycocyamine, la N,N-dichloroglycine.

On peut également utiliser au même effet le complexe de phosphate trisodique hydraté et d'hypochlorite de sodium de formule $n(Na_3PO_4, xH_2O) NaOCl$, où $n = 4$ à $7$ et $x = 11$ à $12$, que l'on désignera dans la suite par P.T.C.

La quantité de réducteur qui doit être utilisée pour réduire le peroxyde d'hydrogène suivant une cinétique favorable varie de 0,02 % à 6 %, et de préférence d'environ 0,04 % à 1 %.

Selon une caractéristique importante de l'invention, le procédé consiste à faire intervenir les deux composés de manière séquentielle, grâce à une présentation structurelle spécifique des compositions de l'invention telle que par exemple comprimé multicouches, comprimé à plusieurs noyaux, l'un des composés étant isolé de l'autre par un enrobage approprié, ou par une présentation séparée des deux composés dans un conteneur différent, par exemple double sachet ou système mécanique (récipient basculant).

L'invention vise donc également des compositions pour la stérilisation et le nettoyage des lentilles de contact, du type comprenant un premier agent, de caractère oxydant, ayant pour effet de détruire les germes présents sur la lentille, et un second agent, de caractère réducteur, ayant pour effet, par réaction avec le premier agent, de donner naissance à des sous-produits de dégradation réciproque inoffensifs pour l'oeil, l'un des deux agents ayant pour effet additionnel de détruire les protéines en vue d'éliminer le voile troublant la transparence de la lentille, tandis que l'autre agent possède lui-même une action bactéricide s'exerçant sur des germes dont certains au moins sont différents de ceux sensibles au premier agent, la nature et les proportions de ces agents étant celles déjà indiquées plus haut, et ces agents intervenant de manière séquentielle, en fonction de la présentation spécifique précitée.

Ces compositions sont mises en oeuvre de la manière suivante. Les lentilles sont disposées par l'utilisateur dans un récipient quelconque, par exemple l'étui des lentilles, dans lequel on a préalablement disposé une quantité d'eau, fonction du volume du récipient et dans lequel on ajoute une quantité suffisante de la composition de l'invention. Les lentilles sont mises en contact avec la composition pendant une durée fonction du système particulier utilisé, à température ambiante. Le système assure toute sécurité, car les lentilles peuvent y séjourner plusieurs jours sans danger, les produits de décomposition du système oxydo-réducteur étant inoffensifs pour l'oeil.

Avantageusement, les compositions selon l'invention renferment des additifs tels que la solution, dans laquelle baignent les lentilles après traitement, soit isotonique et iso-pH (7,0.7,6).

A la fin de cette opération de nettoyage et de décontamination, le porteur peut directement reposer ses lentilles convenablement traitées sur les yeux, sans aucun traitement ou rinçage ultérieur.

Le procédé décrit ci-dessus permet de nettoyer et de décontaminer des lentilles quotidiennement en les maintenant dans leur état initial. Il permet de le faire dans une situation de sécurité totale pour l'utilisateur. Les produits et mode d'emploi du produit sont tels que l'utilisateur ne puisse commettre aucune erreur opératoire, la décontamination et le nettoyage se faisant en une opération unique.

Les substances qui sont utilisées dans la présente invention, à savoir les oxydants et les réducteurs, sont choisis de manière à ce qu'ils n'affectent pas les caractéristiques physiques de la lentille et à ne pas être toxiques pour les yeux de l'utilisateur des lentilles, ni à générer de tels produits toxiques.

La solution finale après nettoyage et décontamination est isotonique et iso-pH aux larmes pour les raisons de tolérance des tissus oculaires mis en contact avec les produits et pour des raisons de compatibilité avec le polymère de la lentille.

On pourra incorporer aux substances actives décrites plus haut des excipients tampons, des agents de stabilisation convenables comme le benzoate de sodium, l'édétate disodique, le chlorure ou le citrate de sodium, le chlorure ou le citrate de potassium, des phosphates et bicarbonates de sodium et potassium.

On a précisé précédemment que la mise en contact retardée de deux composés pouvait s'opérer soit à l'intérieur de présentations spécifiques (comprimés à noyaux multiples ou multi-couches), soit par une séparation physique (plusieurs sachets par exemple, ou produits portés par un support poreux en papier).

Dans le deuxième cas, il n'y a pas de problème particulier à résoudre, les composés ne pouvant pas venir en contact avant leur utilisation.

Dans le premier cas, par contre, il est impératif que les présentations externes garantissent une bonne conservation des produits, réactifs entre eux, dans le temps.

A titre d'exemple, des excipients non toxiques pour l'oeil appropriés permettant d'une part la libération retardée du réducteur et permettant d'autre part d'éviter l'incompatibilité chimique entre l'oxydant et le réducteur durant la conservation du produit, seront employés en combinaison avec les compositions de l'invention.

Ainsi le traitement pourra être réalisé avec un comprimé à double noyau, ou comprimé multi-couches avec enrobage du réducteur, libérant de manière séquentielle programmée dans le temps:

- le composé oxydant
- puis le produit réducteur de neutralisation, le tout dans un même milieu, rendu isotonique par les excipients du comprimé, ou inversement le composé réducteur puis le composé oxydant.

Comme produit d'enrobage permettant d'isoler soit les grains de l'un des deux composés dans une composition granulée unique, soit le noyau central d'un comprimé comportant le premier composé enrobé à l'intérieur du second composé, on peut citer le sel de polyméthylméthacrylate soluble à pH 7, commercialisé par la firme RÖHM PHARMA sous la marque "EUDRAGIT L-100" (marque déposée).

L'action des compositions selon l'invention peut être renforcée si on utilise un mélange effervescent comportant, outre les composés décrits ci-dessus, un produit dégageant, après mise en solution, du gaz carbonique dont l'action mécanique est nettoyante. Ce produit pourra être choisi par exemple, de maniere non limitative, dans le groupe comprenant le bicarbonate de sodium, l'acide citrique, le bicarbonate de sodium et le carbonate glycine de sodium (CGS), le citrate de sodium monosodique, le phosphate monosodique.

Exemple I. Des compositions conformes à l'invention sont par exemple les suivantes:

|  | Composition1 | Composition2 | Composition3 |
|---|---|---|---|
| – peroxyde d'urée | 1,5 mg | 1,05 mg | 2,25 mg |
| – P.T.C. enrobé | 24,0 mg | 16,8 mg | 36,0 mg |
| – Citrate disodique anhydre | 30,0 mg | 21,0 mg | 45,0 mg |
| – CGS | 10,0 mg | 7,0 mg | 15,0 mg |
| – Chlorure de sodium | 70,0 mg | 49,0 mg | 105,0 mg |

Ces compositions sont à dissoudre respectivement dans 10 ml, 7 ml et 15 ml d'eau.

Cette formulation évite la possibilité de réaction entre le composé oxydant, et le phosphate trisodique chloré (PTC) dans un environnement chargé d'humidité.

Cette difficulté a été détournée en empêchant le contact direct entre l'oxydant et le réducteur par utilisation de polymères d'enrobage du type acide méthacrylique et ester méthylique d'acide méthacrylique. Ce polymère aura donc une double fonction:

1) empêcher la libération immédiate du réducteur en solution en la différant par rapport au peroxyde d'urée,

2) empêcher l'incompatibilité physico-chimique d'une part entre l'oxydant et le réducteur, d'autre part entre les produits réactifs et certains excipients.

Ces mélanges se présentent sous forme de poudre fine, non collante. Leur temps de dissolution est inférieur à 30 minutes. Ils produisent dans l'eau une légère effervescence due au citrate disodique et au C.G.S. (carbonate glycine de sodium).

Exemple II. Pour stabiliser encore plus le comprimé, et éviter les éventuelles incompatibilités:
- PTC / peroxyde d'urée,
- PTC / acides
- CGS / acide citrique
om réalise avantageusement un comprimé tri-couches.

A cet effet, on a recherché d'une part un comprimé destiné à être dissous dans 7 ml d'eau purifiée, d'autre part un comprimé tri-couches destiné à être dissous dans 7 ml d eau physiologique.

**a) Comprimé dissous dans l'eau purifiée.**

**La formulation retenue est la suivante :**

| 1ère couche | : Peroxyde d'urée | 1,05 mg |
|---|---|---|
|  | Acide citrique anhydre | 5,6 mg |
|  | Chlorure de sodium | 20,0 mg |
|  | lactose | 20,0 mg |
| 2ème couche | : lactose | 23,0 mg |
|  | CGS | 7,0 mg |
| 3ème couche | : PTC enrobé | 16,8 mg |
|  | Chlorure de sodium | 29,0 mg |
|  |  | 122,45 mg |

Chaque couche est lubrifiée par du benzoate de sodium, du PEG 6000 et des amidons hydrolysés.

Ce comprimé est à dissoudre dans 7 ml d'eau purifiée.

L'effervescence de ces comprimés tri-couches peut être obtenue en mettant le CGS dans la couche centrale (on a alors un effet retard accentué de la 3ème couche) ou en l'incorporant dans la couche contenant le P.T.C. (dans ce cas, on augmente la quantité de lactose dans la 2ème couche).

b) <u>comprimé dissouç dans l'eau physiologique (7ml)</u>

Une formulation avantageuse est la suivante :

| | | | |
|---|---|---|---|
| <u>1ère couche</u> | : | Peroxyde d'urée | 1,05 mg |
| | | Acide citrique anhydre | 5,6 mg |
| | | Lactose | 40,0 mg |
| <u>2ème couche</u> | : | Lactose | 30,0 mg |
| | | CGS | 7,0 mg |
| <u>3ème couche</u> | : | PTC enrobé | 16,8 mg |
| | | Lactose | 25,0 mg |

Exemple III. Cet exemple montre que le prétraitement au peroxyde d'urée selon l'invention détruit rétroactivement l'hypochlorite ou l'acide hypochloreux. Le système rédox à libération programmée, ou le réducteur est libéré en solution un certain temps après l'oxydant pour permettre à ce dernier d'agir, est de préférence sous la forme d'un comprimé bi- ou tri-couches.

L'hypochlorite libéré à partir du phosphate trisodique chloré est lui-même oxydé par l'eau oxygénée produite par le peroxyde d'urée. La cinétique de neutralisation du $Cl_2$ libre montre qu'après 5 min de contact entre l'oxydant et le réducteur, la moitié du $Cl_2$ libéré est déjà détruite par le peroxyde d'urée. Après 1 heure, il ne reste plus que 0,1 mg %, soit 1 ppm, de $Cl_2$ libre dans la solution. Cette quantité de chlore est parfaitement biocompatible.

Exemple IV.

Cet exemple illustre l'efficacité antimicrobienne d'une composition selon l'invention, utilisée sous formé de deux comprimés séparés.

Le comprimé A a la composition suivante :

Peroxyde d'urée enrobé d'EUDRAGIT® L 100 correspondant à peroxyde d'urée pur 1,18 mg

Lactose 50,00 mg

Benzoate de sodium 5,00 mg

Le comprimé B a la composition suivante:

Dichloroisocyanurate de sodium. 2H20 1,00 mg

Phosphate monosodique anhydre 32,00 mg

Phosphate disodique anhydre 50,00 mg

Bicarbonate de sodium 57,00 mg

Lactose 30,00 mg

Benzoate de sodium 15,00 mg

Les deux comprimés sont introduits dans 10 ml de suspension de germes à $10^6$ germes/ml.

Germes utilisés

- Staphylococcus aureus : ATCC 6538 P
- Pseudomonas aeruginosa : ATCC 19429
- Escherichia coli + : ATCC 8739
- Candida albicans : ATCC 10231
- Aspergillus niger : ATCC 16404

La détermination de l'efficacité antimicrobienne des comprimés est résumée dans le tableau qui suit.

| | Témoins t = 0 | Essais t = 0 | Essais t = 1h | Essais t = 2h | Témoin t = 18h | Essais t = 18h |
|---|---|---|---|---|---|---|
| Candida albicans | 41.000 | ø | ø | ø | 460.000 | ø |
| Aspergil-lus niger | 200.000 | ø | ø | ø | 200.000 | ø |
| Staphylo-coccus aureus | 4.000.000 | ø | ø | ø | 200.000 | ø |
| Pseudomo-nas aeruginosa | 3.000.000 | ø | ø | ø | 2.700.000 | ø |
| Escherichia coli | 530.000 | ø | ø | ø | 300.000 | ø |

"Poudre A'''
- Peroxyde d'urée 1,5 mg
- Benzoate de sodium 3,5 mg
- Chlorure de sodium 40,0 mg
- Lactose 80,0 mg
"Poudre B"
- P.T.C. 24,0 mg
- Benzoate de sodium 3,5 mg
- Chlorure de sodium 17,0 mg
- Lactose 60,5 mg

La poudre "A" est mise en solution dans 10,0 ml d'eau purifiée contenue dans un récipient adéquat permettant de recevoir les lentilles. Après 10 minutes d'attente on rajoute la poudre "B". On observe une légère effervescence, la solution finale obtenue étant claire et limpide, présentant un pH de 7,3 et un point cryoscopique de -0,56°C.

Le dosage après 15 minutes de mise en solution des poudres démontre une concentration en peroxyde d'hydrogène inférieure à 2 ppm et une concentration en chlore inférieure à 1 ppm.

Essai bactériologique:

La poudre "A" est mise en solution dans 10 ml de suspensions de germes à $10^6$ germes/ml. Après 10 minutes, on rajoute la poudre "B", et on prélève après 1,2 et 5 minutes pour effectuer le dénombrement des germes survivants.

Germes testés :
Escherichia coli ATCC 8739
Pseudomonas aeruginosa ATCC 9027
Staphylococcus aureus ATCC 65380
Résultats:

| | Concentration des germes survivants après | | |
|---|---|---|---|
| Temps de contact(mn) | 1 | 2 | 5 |
| E. coli | $< 10$ | $< 10$ | $< 10$ |
| Ps. aeruginosa | $1,9. 10^4$ | $7.10^2$ | $< 10$ |
| St. aureus | $2.9. 10^4$ | $< 10$ | $< 10$ |

Exemple VI

Cet exemple illustre l'efficacité antimicrobienne de deux compositions selon l'invention utilisées chacune sous forme d'un comprimé unique.

Comprimé A

Noyau interne

| | |
|---|---|
| Peroxyde d'urée | 1,05 mg |
| Acide citrique anhydre | 5,60 mg |
| Chlorure de sodium | 15,00 mg |
| Lactose | 22,15 mg |
| Carboxymethylcellulose, Sel de Sodium | 6,40 mg |
| Benzoate de sodium | 2,40 mg |
| P.E.G. 6000 | 2,40 mg |

| | |
|---|---|
| + Eudragit® L 100(enrobage du noyau) | 1,5 mg |
| Noyau interne de | 56,50 mg |

Couverture externe

| | |
|---|---|
| P.T.C. | 16,80 mg |
| Chlorure de sodium | 40,00 mg |
| Lactose | 48,70 mg |
| Carboxymethyl amidon sodique | 6,00 mg |
| Benzoate de sodium | 8,50 mg |
| P.E.G. 6000 | 8,50 mg |
| Couverture externe de | 128,50 mg |

Comprimé B

Comprimé double noyau biconvexe, de couleur blanche $\phi$ 7,5 mm, Épaisseur : 4 mm.

Noyau interne enrobé

$\phi$ : 5,0 mm, Épaisseur : 2,5 mm

Composition
Noyau interne

| Matières premières | Quantité unitaire |
|---|---|
| Peroxyde d'urée enrobé | 3,16 mg |
| Lactose | 41,09 mg |
| Benzoate de sodium | 5,00 mg |
| Carboxymethylamidon sodique | 1,25 mg |
| Eudragit L 100 | |
| (Enrobage) | 1,50 mg |
| pour un comprimé de 52,00 mg | |

Couverture

| Matières premières | Quantité unitaire |
|---|---|
| Dichloroisocyanurate de Na.2H$_2$O | 1,00 mg |
| Phosphate disodique anhydre | 63,50 mg |
| Phosphate monosodique anhydre | 26,50 mg |
| Bicarbonate de sodium | 40,00 mg |
| Chlorure de sodium | 15,00 mg |
| Benzoate de sodium | 8,00 mg |
| P.E.G. 6000 | 15,00 mg |
| Pour une couverture de 169,00 mg | |

→ Comprimé double noyau de 221,00 mg

Les comprimés A et B sont destinés à être dissous dans 10 ml d'eau.

Ces comprimés ainsi obtenus présentent une surface très régulière, non collante. Leur temps de dissolution est inférieur à 20 minutes. Ils produisent dans l'eau une effervescence dûe au phosphate monosodique et bicarbonate de sodium.

La solution obtenue est limpide et incolore, donnant un pH de 7. 2 et un point cryoscopique de -0,57° C.

Essai bactériologique :

Pour démontrer l'efficacité antimicrobienne du comprimé B, on prépare des suspensions à $10^6$ germes/ml et l'on met un comprimé en contact avec 10 ml de suspension. On prélève après dissolution du comprimé formé, après 1,2 et 18 heures, et on dénombre les germes survivants :

Microorganismes utilisés :
- Candida albicans ATCC 10.231
- Aspergillus niger ATCC 16404
- Escherichia coli ATCC 8739
- Pseudomonas aeruginosa ATCC 19429
- Staphylococcus aureus ATCC 6538

8

0 124 461

Résultats : Concentration des micro-organismes après dénombrement.

| Organismes | T = 0 dans la solution physiolo-gique témoin | disso-lution du com-primé | Conc. des org/ml dans le produit, après (heures) | | |
|---|---|---|---|---|---|
| | | | 1 h | 2 h | 18 h |
| C. albicans (ATCC 10231) | 370.000 | O | O | O | O |
| Asp. niger (ATCC 16404) | 100.000 | O | O | O | O |
| St. aureus (ATCC 6538) | 2.600.000 | O | O | O | O |
| Ps. aeruginosa (ATCC 14429) | 1.540.000 | O | O | O | O |
| E.coli (ATCC 8937) | 1.970.000 | O | O | O | O |

Exemple VII

Cet exemple illustre l'efficacité antibactérienne d'une composition selon l'invention utilisée sous forme d'une poudre unique, dans laquelle les grains de l'un des deux agents sont enrobés d'EUDRAGIT® L 100.

Compositions

| | I | II |
|---|---|---|
| Peroxyde d'urée | 2,0 mg | 2,0 mg |
| Dichloroisocyanurate de sodium enrobé (EUDRAGIT® L 100) | 2,0 mg | 2,0 mg |
| Phosphate disodique anhydre | 60,1 mg | 120,2 mg |
| Phosphate monosodique anhydre | 19,7 mg | 39,4 mg |
| Lactose | 92,0 mg | 92,0 mg |
| Benzoate de sodium | 10,0 mg | 10,0 mg |
| Chlorure de sodium | 44,2 mg | 6,0 mg |

Ces compositions sont à dissoudre dans 10 ml d'eau et amènent après dissolution à une solution de pH 7,18 et point cryoscopique -0,56°C pour la composition 1, et une solution de pH 7,0 et point cryoscopique de -0,57°C pour la composition II.

Cette formulation évite la possibilité de réaction entre le peroxyde d'urée et la dichloroisocyanurate de sodium et permet une libération retardée du DCCNa en solution.

9

Les mélanges ainsi obtenus se présentent sous forme de poudres fines, non collantes.

Essai bactériologique :

Les tests sont conduits sur la composition II ci-dessus.

A 10 ml de suspension de germes à $10^8$ germes/ml, on ajoute au temps 0 la quantité adéquate de la poudre testée. Après 1,3,5 et 10 minutes de mise en contact, les germes survivants sont dénombrés.

Germes testés :

Escherichia Coli ATCC 8739

Pseudomonas aeruginosa ATCC 9027

Staphylococcus aureus ATCC 65380

Résultats :

Concentration des micro-organismes survivants en fonction du temps de contact et des dilutions effectuées lors du dénombrement.

a) <u>Escherichia coli</u>

| Dilutions / Temps de contact | 1/10 e | 1/100 e | 1/1000 e | 1/10 000 e |
|---|---|---|---|---|
| 1 minute | < 10 | < 10 | < 10 | < 10 |
| 3 minutes | < 10 | contaminé | contaminé | < 10 |
| 5 minutes | < 10 | < 10 | < 10 | < 10 |
| 10 minutes | < 10 | < 10 | < 10 | < 10 |

b) <u>Staphylococcus aureus</u>

| Dilutions / Temps de contact | 1/10 e | 1/100 e | 1/1000 e | 1/10 000 e |
|---|---|---|---|---|
| 1 minute | < 10 | < 10 | < 10 | < 10 |
| 3 minutes | < 10 | < 10 | < 10 | < 10 |
| 5 minutes | < 10 | < 10 | < 10 | < 10 |
| 10 minutes | < 10 | < 10 | < 10 | < 10 |

c) <u>Pseudomonas aeruginosa</u>

| Dilutions / Temps de contact | 1/10 e | 1/100 e | 1/1000 e | 1/10 000 e |
|---|---|---|---|---|
| 1 minute | < 10 | < 10 | < 10 | < 10 |
| 3 minutes | < 10 | < 10 | < 10 | < 10 |
| 5 minutes | < 10 | < 10 | < 10 | < 10 |
| 10 minutes | < 10 | < 10 | < 10 | < 10 |

<u>Exemple VIII</u>

On prépare trois solutions dans du sérum physiologique. La solution 1 est préparée à partir de l'association des deux substances, la solution 2 ne contient que du peroxyde d'urée, la solution 3 ne contient que du PTC. On ajoute à chacune des solutions indiquées trois types de bactéries différentes à des concentrations de $10^5$ à $10^6$ germes/ml. On observe le nombre de bactéries vivantes par culture sur milieu nutritif solide après 1h 30 de contact. Les résultats obtenus sont repris ci-après.

Expérience 1.
La formule testée est la formule complète : Peroxyde d'urée et PTC mis au même moment en contact avec les bactéries. On mesure la concentration des micro-organismes après dénombrement.

| ORGANISMES MICROBIENS | Nombre de germes par ml de produit à l'inoculation | Nombre de germes par ml de produits après 1h30 de contact. |
|---|---|---|
| E.coli | 780.000 | 2900 |
| Ps. aeruginosa | 590.000 | 3400 |
| St. aureus | 1 080 000 | 4000 |

Expérience 2.
Peroxyde d'urée et PTC mis l'un après l'autre en contact : Peroxyde d'urée + inoculum en contact pendant 15 min, puis restant de la formule.

| ORGANISMES MICROBIENS | Nombre de germes par ml de produit à l'inoculation | Nombre de germes par ml de produit après 1h30 de contact |
|---|---|---|
| E.coli | 980 000 | 3000 |
| Ps. aeruginosa | 770 000 | 0 |
| St. aureus | 890 000 | 190 |

Expérience 3.
PTC + inoculum en contact pendant 15 min, puis restant de la formule.

| ORGANISMES MICROBIENS | Nombre de germes par ml de produit à l'inoculation | Nombre de germes par ml de produit après 1h 30 de contact |
|---|---|---|
| E.coli | 980 000 | 90 |
| Ps. aeruginosa | 770 000 | 660 |
| St.aureus | 890.000 | 0 |

Dans l'expérience 1, il n'y a pas d'effet retard : les deux substances sont mises en contact immédiatement. Dans l'expérience 2, on simule l'effet retard en faisant agir d'abord le peroxyde d'urée pendant 15 min, puis le reste de la composition.

L'expérience 3 est identique, le PTC étant mis en contact des germes d'abord puis le reste de la composition.

Ces résultats mettent en évidence l'effet synergique antibactérien de l'association du peroxyde d'urée et du PTC particulièrement utile lorsque les deux substances sont mises en contact des bactéries l'une après l'autre. L'activité du système redox complet est significativement supérieure à celle de chacun des composés pris séparément.

Enfin en ce qui concerne l'efficacité des compositions selon l'invention en tant que produits de nettoyage des lentilles, éliminant les dépôts protéiniques responsables du boile qui se forme durant le port des lentilles, le test utilisé consiste à provoquer l'encrassage de lentilles par immersion pendant 24 à 48 h dans une solution isotonique de chlorure de sodium à 10 % de serum bovin atténué. On constate la formation d'un voile. Après traitement des lentilles voilées au moyen des compositions selon l'inventiom, et notamment par application de celles faisant l'objet des exemples IV et VI, sans neutralisation ni rinçage, on constate subjectivement la disparition de ce voile, et le retour des lentilles à une transparence parfaite. Le test a été effectué à la fois sur des lentilles souples et sur des lentilles dures.

Afin de démontrer la bonne tolérance et l'efficacité du nettoyage - Six lapins Albinos sont équipés en lentilles de contact souples hydrophiles. Une des lentilles est traitée quotidiennement au moyen des compositions selon l'invention, et notamment par application de celle faisant l'objet de l'exemple VI ; l'autre lentille, servant de témoin, est traitée de manière classique en employant une solution de nettoyage et conservation contenant un surfactif et des anticeptiques.

Après 25 jours de traitement quotidien, on a pu constater que les lentilles traitées par la composition selon l'invention, contrairement aux lentilles témoins, ne présentaient aucune trace de dépôts; démontrant ainsi que la composition testée permet outre un nettoyage efficace, la prévention d'une éventuelle accumulation de dépôts au niveau des lentilles.

De plus durant les 25 jours de test, aucune reaction d'intolérance ni variation anormale de l'état des cornées n'a été notée.

On suppose que l'efficacité des compositions selon l'invention, à la fois comme compositions anti-microbiennes et comme agents de nettoyage provient de l'action de l'oxygène naissant qui se dégage au moment de leur utilisation, qui exerce une très forte action oxydante, particulièrement efficace sur les germes anaerobies et sur les dépôts protéiniques, tandis que l'agent réducteur et notamment le dichlorocyanurate de sodium exerce son action antibactérienne propre sur toutes les espèces de germes.

## Revendications

1. Procédé pour la stérilisation et le nettoyage des lentilles de contact du type consistant à traiter la lentille par un premier agent, de caractère oxydant, ayant pour effet de détériorer les germes présents sur la lentille, puis par un second agent, de caractère réducteur, donnant naissance par réaction avec le premier agent à des sous-produits de dégradation inoffensifs pour l'oeil, le procédé étant caractérisé en ce que l'agent oxydant est choisi parmi les produits capables de libérer de l'eau oxygénée in situ alors que l'agent réducteur est choisi parmi les produits aptes à générer de l'acide hypochloreux en solution aqueuse et stables à l'état sec, les proportions respectives d'agent oxydant et d'agent réducteur étant d'envrion 0,005 % à 5 % et 0,02 % à 6 % en poids des substances mises en jeu.

2. Procédé selon la revendication 1, caractérisé en ce que l'agent oxydant est choisi dans le groupe: peroxyde d'urée, perphosphates alcalins, percarbonates alcalins, perborates alcalins et persulfates alcalins.

3. Procédé selon l'une quelconque des revendications 1 ou 2, caractérisé en ce que l'agent réducteur est constitué par un hypochlorite minéral ou organique, un complexe de phosphate trisodique hydraté et d'hypochlorite de sodium, du dichloroisocyanurate de sodium.

4. Procédé selon l'une quelconque des revendications 1 à 3, caractérisé en ce que les deux agents sont utilisés sous forme de composition dosée solide, contenant des excipients connus en soi, permettant de libérer successivement en milieu aqueux le premier puis le second agent.

5.Compositions pour la stérilisation et le nettoyage de lentilles de contact obtenues selon le procédé de l'une quelconque des revendications 1 à 4, caractérisées en ce que chaque agent est présent sous forme de poudre ou micro-granules en deux conditionnements séparés qui sont ensuite mis en solution aqueuse pour libérer les produits actifs.

6. Compositions obtenues selon le procédé de l'une quelconque des revendications 1 à 4, caractérisées en ce que l'un des deux agents est isolé de l'autre par une matière d'enrobage soluble dans l'eau à pH 7 environ, les deux agents étant combinés, en comprimé à double noyau ou poudre mixte, dans une forme d'utilisation unique.

7. Compositions selon la revendication 6, caractérisée en ce que la matière d'enrobage est constituée par un sel soluble de polyméthacrylate de méthyle.

## Patentansprüche

1. Verfahren zur Sterilisierung und Reinigung von Kontaktlinsen, bei welchem die Linse mit einem ersten Mittel mit oxidierenden Eigenschaften, das die Zerstörung der auf der Linse vorhandenen Keime zum Zweck hat, dann mit einem zweiten Mittel mit reduzierenden Eigenschaften, das durch Reaktion mit dem ersten Mittel für das Auge unschädliche Abbauprodukte entstehen läßt, behandelt wird,
dadurch gekennzeichnet,
daß das Oxidationsmittel ausgewählt wird aus den zur Freisetzung von Wasserstoffperoxid in situ befähigten Stoffen und das Reduktionsmittel ausgewählt wird aus den zur Bildung von unterchloriger Säure in wässeriger Lösung befähigten, in trokkenem Zustand stabilen Produkten, wobei die jeweiligen Anteile an Oxidations- und Reduktionsmittel bei etwa 0,005 % bis 5 % und 0,02% bis 6 %, bezogen auf das Gewicht der eingesetzten Stoffe liegen.

2. Verfahren nach Anspruch 1,
dadurch gekennzeichnet,
daß das Oxidationsmittel ausgewählt wird aus der Gruppe Harnstoffperoxid, Alkaliperphosphate, Alkalipercarbonate, Alkaliperborate und Alkalipersulfate.

3. Verfahren nach irgendeinem der Ansprüche 1 oder 2,
dadurch gekennzeichnet,
daß das Reduktionsmittel aus einem mineralischen oder organischen Hypochlorit, einem Komplex aus hydratisiertem Trinatriumphosphat und Natriumhypochlorit, aus Natriumdichlorisocyanurat besteht.

4. Verfahren nach irgendeinem der Ansprüche 1 bis 3,
dadurch gekennzeichnet,
daß die beiden Mittel in Form einer festen dosierten Form verwendet werden, die an sich bekannte Trägerstoffe enthält und es gestattet, in wässerigem Medium nacheinander das erste und dann das zweite Mittel freizusetzen.

5. Zusammensetzungen zur Sterilisierung und Reinigung von Kontaktlinsen, erhalten nach dem Verfahren nach irgendeinem der Ansprüche 1 bis 4,
dadurch gekennzeichnet,
daß jedes Mittel in Form eines Pulvers oder Mikrogranulats in zwei getrennten Zubereitungen vorliegt, die dann in wässerige Lösung übergeführt werden, um die Wirkstoffe freizusetzen.

6. Zusammensetzungen, erhalten nach dem Verfahren nach irgendeinem der Ansprüche 1 bis 4,
dadurch gekennzeichnet,
daß eines der beiden Mittel vom anderen durch eine Hüllsubstanz getrennt ist, die in Wasser vom pH-Wert etwa 7 löslich ist, und daß die beiden Mittel in einer Doppelkerntablette oder als gemischtes Pulver in einer einzigen Anwendungsform vereint sind.

7. Zusammensetzungen nach Anspruch 6,
dadurch gekennzeichnet,
daß das Hüllmaterial aus einem löslichen Salz von Polymethylmethacrylat besteht.

**Claims**

1. Process for the sterilization and the cleaning of contact lenses of type consisting in treating the lens by a first agent, with oxidizing feature, having the effect to destroy the germs that are presents on the lens, then by a second agent, with reducing feature, having the effect by reaction with the first agent to generate degradation by products that are harmless for eyes, the process being characterized in that the oxidizing agent is selected amongst the products able to discharge in situ hydrogen peroxide while the reducing agent is selected amongts the products able to generate hypochlorous acid in aqueous solution and stable in the dry state, the respective proportions of oxidizing agent and of reducing agent being about 0,005 % to 5 % and about 0,02 % to 6 % with regard to weight of the involved compositions.

2. Process according the claim 1, whereas the oxidizing agent is selected in the group constituted by: peroxide of alkaline perphosphates, alkaline percarbonates, alkaline perborates and alkaline persulfates.

3. Process according any one of the claims 1 or 2, whereas the reducing agent is selected in the group consisting of : a mineral or organic hypochlorite, a complex of hydrated trisodium phosphate and sodium hypochlorite, sodium dichloroisocyanurate.

4. Process according any one of the claims 1 to 3, whereas the two agents are employed under the form of a solid dosed composition displayed with known excipients, so as to determine, in aqueous medium, sequentially the freeing of the first and then of the second agent.

5. Compositions for the sterilization and cleaning of contact lenses, such as obtained by the process of any one of the claims 1 to 4, whereas each agent is present under the form of a powder or of micro-granules in two separated packagings which are then put in aqueous solution to liberate the active products.

6. Compositions obtained by the process of any one of the claims 1 to 4, whereas one of the two agents is isolated from the other by a coating product in water soluble at about pH 7, the two agents being associated in a single form of utilization such as a double nucleous tablet or a mixed powder.

7. Compositions according the claim 6, whereas the coating product is a soluble salt of polymethylmethacrylate.